# EUROPÄISCHE PATENTSCHRIFT

(11) **EP 3 536 369 B1**
(45) Veröffentlichungstag und Bekanntmachung des Hinweises auf die Patenterteilung: **24.07.2024**
(21) Anmeldenummer: 19159730.1
(22) Anmeldetag: 27.02.2019
(51) Int. Cl.: A61M 16/00, A61M 16/08, A61M 16/12, A61M 16/20, A61M 16/10

(54) **BEATMUNGSGERÄT MIT SCHALTVENTIL**
RESPIRATORY APPARATUS WITH SWITCHING VALVE
APPAREIL RESPIRATOIRE À SOUPAPE DE COMMANDE

(30) Priorität: 08.03.2018 DE 102018001888
(43) Veröffentlichungstag der Anmeldung: 11.09.2019
(60) Teilanmeldung: 24178290.3
(73) Patentinhaber: Löwenstein Medical Technology S.A., 2557 Luxembourg (LU)
(72) Erfinder: Adametz, Benjamin, 22609 Hamburg (DE); Göbel, Christof, 22457 Hamburg (DE)
(74) Vertreter: Marx, Thomas

(56) Entgegenhaltungen:
- WO-A1-2017/025178
- WO-A2-2012/085792
- CN-A- 105 617 527
- CN-A- 107 308 531
- DE-A1- 102009 025 327
- GB-A- 2 335 604
- US-A- 5 909 731
- US-A1- 2010 078 017
- US-A1- 2010 319 691
- US-A1- 2012 145 156
- US-B2- 8 893 718

## Beschreibung

Die vorliegende Erfindung betrifft ein Beatmungsgerät mit einem Geräteeingang und einem Geräteausgang und einem Atemgasweg zwischen Geräteeingang und Geräteausgang, wobei im Atemgasweg ein Atemgasantrieb, ein Rückschlagventil und ein Schaltventil angeordnet sind.

Beatmungsgeräte werden für die Therapie respiratorischer Störungen eingesetzt, dabei können die Beatmungsgeräte in der nicht- invasiven und invasiven Beatmung, sowohl innerklinisch als auch außerklinisch, verwendet werden.

Bei einer Beatmung eines Patienten kann in der Regel ein Beatmungsgerät mit einem inspiratorischen Zweig für den Atemgasfluss und optional mit einem Zweig für den exspiratorischen Atemgasfluss eingesetzt werden. Der Zweig für den exspiratorischen Atemgasfluss ermöglicht die Ausatmung/Exspiration eines Atemgases durch den Patienten, während der Zweig für den inspiratorischen Atemgasfluss den Patienten mit Atemgas versorgt.

Die CN107308531 offenbart ein Ventil mit einem Rückschlagventil und ein Beatmungsgerät mit einem solchen Ventil. Gemäß der CN107308531 regelt das Ventil den inspiratorischen Druck. Zudem ist das Ventil eingerichtet, eine exspiratorische Rückatmung des Patienten in das Beatmungsgerät zu verhindern.

Die CN105617527 offenbart ein Ausatemventil, zur Verwendung mit einem Beatmungsgerät, mit einem Bypass zur Umgebungsluft. Der Bypass öffnet bei einem Ausfall des Beatmungsgeräts und ermöglicht dem Patienten eine eigenständige Atmung. Die Aufgabe der CN105617527 ist, dass Ausatemluft in die Umgebung abgeleitet wird und dass somit nicht in das Beatmungsgerät zurück geatmet wird.

Die US2012145156 offenbart ein Sprechventil zur Verwendung bei beatmeten Patienten, die ein Tracheostoma haben und daher unter der Beatmung nicht sprechen können.

Die GB2335604 offenbart ein Anästhesiegerät mit einem Atemgaskreislauf und einem CO2-Absorber. Das Anästhesiegerät ist eingerichtet, möglichst wenig Narkosegase zu verlieren und entzieht dem exspiratorischen Atemgas daher CO2 und führt Sauerstoff hinzu.

Die US8893718 offenbart ein Anästhesiegerät mit einer Beatmungseinheit. Die Beatmungseinheit weist einen inspiratorischen Zweig auf und einen exspiratorischen Zweig auf. Beide Zweige sind unidirektional. Im Bereich einer patientennahen Zweigstelle ist ein Druckentlastungsventil angeordnet.

Die US5909731 offenbart ein Sicherheitsventil für die Beatmung, welches den Beatmungsdruck begrenzt.

Die WO2017025178 offenbart ein Anästhesiegerät mit einer Beatmungseinheit, die einen beweglichen Balg aufweist. Der Balg trennt dabei das Atemgas vom Druckgas, welches den Balg antreibt.

Die US2010319691 offenbart ein Druckregelsystem, welches einen kontrollierten Überdruck und Unterdruck anlegt.

Die DE102009025327 offenbart ein Beatmungsgerät, welches Atemgas zur Kühlung von Geräteteilen liefert.

Die WO2012085792 offenbart ein Beatmungssystem, welches kontrollierten Überdruck und Unterdruck anlegt und einen inspiratorischen und einen exspiratorischen Zweig aufweist.

Die US2010078017 offenbart ein Beatmungssystem mit drahtloser Kommunikation von Beatmungsdaten.

Bei den im Stand der Technik bekannten Beatmungsgeräten kann es zu sowohl zu Blockaden/Störungen im exspiratorischen Zweig als auch im inspiratorischen Zweig des Beatmungsgerätes kommen.

Eine Blockade/Störung im exspiratorischen Zweig kann beispielsweise durch Materialeintrag, wie beispielsweise Medikamente oder Sekret, verursacht werden. Blockaden/Störungen im exspiratorischen Zweig können einen Patienten an einer Ausatmung von Atemgas hindern.

Eine Blockade/Störung des exspiratorischen Zweiges kann beispielsweise dazu führen, dass der Atemwegsdruck in der Exspiration nicht auf das angestrebte Druckniveau gesenkt werden kann, wodurch ein Patient nicht ausatmen kann. Ein anhaltender erhöhter Atemgasdruck in Atemwegen des Patienten kann schädliche Folgen haben. Beispielsweise kann es zu einem gefährlichen Anstieg des intrathorakalen Drucks kommen, der zu einem verminderten venösen Rückfluss, verminderter Herzleistung und schließlich zu einem Abfall des arteriellen Blutdrucks führt.

Neben der Problematik einer Blockade/Störung im exspiratorischen Zweig des Beatmungsgerätes kann es auch zu einer Blockade/Störung im inspiratorischen Zweig des Beatmungsgerätes kommen. Eine Blockade/Störung im inspiratorischen Zweig kann beispielsweise durch einen Ausfall des Atemgasantriebs hervorgerufen werden. Blockaden/Störungen im inspiratorischen Zweig können Patienten an einer Einatmung hindern oder diese zumindest erschweren.

Die im Stand der Technik bekannten Beatmungsgeräte weisen somit den Nachteil auf, dass sie auch bei vorhandenen Sicherungsvorrichtungen nicht ausreichend gegen Blockaden, Störungen oder Ausfälle gesichert sind, sodass eine Inspiration und/oder Exspiration von Atemgas nicht sichergestellt werden kann.

Es ist daher Aufgabe der vorliegenden Erfindung, ein Beatmungsgerät zur Verfügung zu stellen, das eine Sicherung einer Exspiration von Atemgas bei einer Störung / Blockade eines exspiratorischen Zweiges des Beatmungsgerätes sicherstellt.

Gegenstand der vorliegenden Erfindung ist ein Beatmungsgerät nach Anspruch 1. Die Unteransprüche definieren Ausführungsbeispiele der Erfindung. Ein Atemgasantrieb kann ein Gebläse oder eine Druckquelle, insbesondere eine Sauerstoff- oder Luftdruckquelle, sein. Ein Schaltventil kann elektromechanisch ausgebildet sein. Beispielsweise kann ein Schaltventil ein Pneumatikventil mit einem Magentventil sein bzw. ein Bypass mit einem Magnetventil. Ein Schaltventil kann ein 3/2-Wegeventil, ein Pneumatikventil oder ein Membranventil sein.

In der Regel kann ausgeatmetes Atemgas des Patienten über den exspiratorischen Atemgasweg des Beatmungsgerätes abgeführt werden. Eine Rückführung des Atemgases über den inspiratorischen Atemgasweg kann somit verhindert werden.

Das Rückschlagventil verhindert eine ungewollte Kontamination des Atemgasantriebs mit verunreinigtem Atemgas des Patienten bei einem normalen Betrieb des Beatmungsgerätes. Durch das umfasste Schaltventil kann, bei einer Blockade/Störung des exspiratorischen Atemgaswegs, zumindest zeitweise das Rückschlagventil umgangen werden. Dies bietet den Vorteil, dass bei einer Blockade des exspiratorischen Atemgasweges Atemgas des Patienten über den inspiratorischen Atemgasweg abgeführt werden kann.

In Ausgestaltung ist zwischen dem Rückschlagventil und dem inspiratorischen Geräteausgang eine Flussmesseinrichtung angeordnet. Die Flussmesseinrichtung ist eingerichtet einen Fluss bzw. ein Volumen oder einen Druck des Atemgases im Atemgasweg zu erfassen. Das Schaltventil ist eingerichtet, basierend auf dem erfassten Fluss bzw. Volumen oder Druck des Atemgases öffnenbar oder schließbar zu sein. Dies ermöglicht eine atemgasvolumen- oder atemgasdruckabhängige Schaltung des Schaltventils. Alternativ kann das Schaltventil zeitgesteuert oder patientengetriggert steuerbar sein. Das Schaltventil kann beispielsweise durch ein 3/2-Wegeventil schaltbar sein. Durch die Ausbildung des geschlossenen Bypasses um das Rückschlagventil, kann das Rückschlagventil derart umgangen werden, dass das Atemgas um das Rückschlagventil herum in den selben Atemgasweg eingeleitet wird, von dem es abgezweigt wurde.

In Ausgestaltung ist das mindestens eine Schaltventil eingerichtet, den Bypass zu öffnen und/oder zu schließen, wobei das mindestens eine Schaltventil automatisch oder aktiv schaltbar eingerichtet ist. In der Regel ist das Schaltventil eingerichtet, basierend auf dem ermittelten Fluss bzw. Volumen oder Druck des Atemgases automatisch zu öffnen oder zu schließen. Beispielsweise öffnet das Schaltventil wenn die Flussmesseinrichtung einen höheren Druck über einen bestimmten Zeitraum erfasst.

In Ausgestaltung ist der Atemgasantrieb zwischen dem Rückschlagventil und dem inspiratorischen Geräteausgang angeordnet und eingerichtet,

Atemgas in Richtung des inspiratorischen Geräteausgangs zu fördern. Durch die Anordnung des Atemgasantriebs zwischen dem Rückschlagventil und dem inspiratorischen Geräteausgang befinden sich das Rückschlagventil und das mindestens eine Schaltventil auf der Saugseite des Beatmungsgerätes.

Erfindungsgemäß zweigt der Bypass zu dem Schaltventil zwischen dem inspiratorischen Geräteeingang und dem Rückschlagventil ab und mündet zwischen dem Rückschlagventil und dem Atemgasantrieb wieder in den Atemgasweg. Dies bietet den Vorteil, dass das Rückschlagventil umgangen werden kann. Zudem sind bei einer derartigen Anordnung das mindestens eine Schaltventil und das Rückschlagventil auf der Saugseite des Beatmungsgerätes angeordnet.

In einer weiteren Ausgestaltung ist zusätzlich zu dem Bypass ist ein zweiter inspiratorischer Atemgasweg ausgebildet, der einen zweiten inspiratorischen Geräteeingang und mindestens ein weiteres Rückschlagventil umfasst und vor dem gemeinsamen inspiratorischen Geräteausgang in den ersten inspiratorischen Atemgasweg mündet. Der zweite inspiratorische Atemgasweg ist eingerichtet, bei einer Blockade/Störung des inspiratorischen Atemgaswegs einen separaten Atemgasweg bereitzustellen, über den Atemgas inspirierbar ist. Der zweite inspiratorische Atemgasweg kann einen separaten Atemgasantrieb umfassen. Beispielsweise ist der Atemgasantrieb zwischen dem zweiten Geräteeingang und dem Rückschlagventil des zweiten Atemgaswegs angeordnet. Ferner kann der zweite Atemgasweg eine separate Flussmesseinrichtung umfassen, die eingerichtet ist, den Fluss bzw. das Volumen oder den Druck des Atemgases im zweiten Atemgasweg zu erfassen.

In einer weiteren erfindungsgemäßen Ausgestaltung umfasst der exspiratorische Atemgasweg einen exspiratorischen Geräteeingang und einen exspiratorischen Geräteausgang umfasst. Der exspiratorische Atemgasweg ist eingerichtet, Atemgas des Patienten abzuführen. Der exspiratorische Geräteeingang ist dabei in der Regel patientennah angeordnet, während der exspiratorische Geräteausgang patientenfern ausgebildet ist. Der exspiratorische Atemgasweg ist eingerichtet, Atemgas des Patienten an den exspiratorischen Geräteausgang abzuführen und somit dem Patienten ein Ausatmen zu ermöglichen.

In einer Weiterbildung führt von dem exspiratorischen Geräteeingang der exspiratorische Atemgasweg zu dem exspiratorischen Geräteausgang und in dem exspiratorischen Atemgasweg sind ein weiteres Schaltventil und eine Flussmesseinrichtung angeordnet. Die Flussmesseinrichtung ist in der Regel zwischen dem exspiratorischen Geräteausgang und dem Schaltventil angeordnet. Das Schaltventil ist in der Regel eingerichtet, basierend auf einem festgelegten Zeitintervall oder einem Patiententrigger schaltbar zu sein. Alternativ kann das Schaltventil basierend auf einem durch eine Flussmesseinrichtung gemessenen Fluss bzw. Volumen oder Druck des Atemgases schaltbar sein. Die zwischen dem exspiratorischen Geräteausgang und dem Schaltventil angeordnete Flussmesseinrichtung ist typischerweise eingerichtet, den Fluss bzw. das Volumen oder den Druck des Atemgases zu erfassen und eine Rückmeldung über das durch den Patienten abgegebene Atemgas an das Beatmungsgerät zu ermöglichen.

In einer weiteren Weiterbildung führt zwischen dem exspiratorischen Geräteeingang und dem Schaltventil ein separater exspiratorischer Atemgasweg mit einem weiteren Schaltventil und einer Flussmesseinrichtung zu einem separaten exspiratorischen Geräteausgang.

Der separate exspiratorische Atemgasweg ist eingerichtet, Atemgas des Patienten beispielsweise bei einer Blockade/Störung des exspiratorischen Atemgaswegs abzuführen. Die zwischen dem exspiratorischen Geräteausgang und dem Schaltventil angeordnete Flussmesseinrichtung ist typischerweise eingerichtet, den Fluss bzw. das Volumen oder den Druck des Atemgases zu erfassen und eine Rückmeldung über das durch den Patienten abgegebene Atemgas zu ermöglichen.

In Ausgestaltung ist an den inspiratorischen Geräteausgang ein Schlauchsystem adaptiert, das mit einem ersten Zweig zu einem Patienteninterface führt und vor dem Patienteninterface mit einem zweiten Zweig zu dem exspiratorischen Geräteeingang führt. Optional ist das Schlauchsystem ein Einschlauchsystem. Alternativ kann an den Geräteausgang ein Schlauchsystem adaptiert sein, dass ein Leckageschlauchsystem ist. Durch das Schlauchsystem wird das Patienteninterface mit mindestens einem inspiratorischen Atemgasweg verbunden. Optional wird das Patienteninterface durch das Schlauchsystem mit mindestens einem inspiratorischen Atemgasweg und mindestens einem exspiratorischen Atemgasweg verbunden.

Im Folgenden werden anhand von stark vereinfachten schematischen Darstellungen von Ausführungsbeispielen näher erläutert. Ein erfindungsgemäßes Ausführungspeispiel stellt ein Beatmungsgerät nach Fig. 2, 4 oder 6 mit einem exspiratorischen Atemgasweg 16c nach Fig. 7 oder 8 dar. Es zeigt:

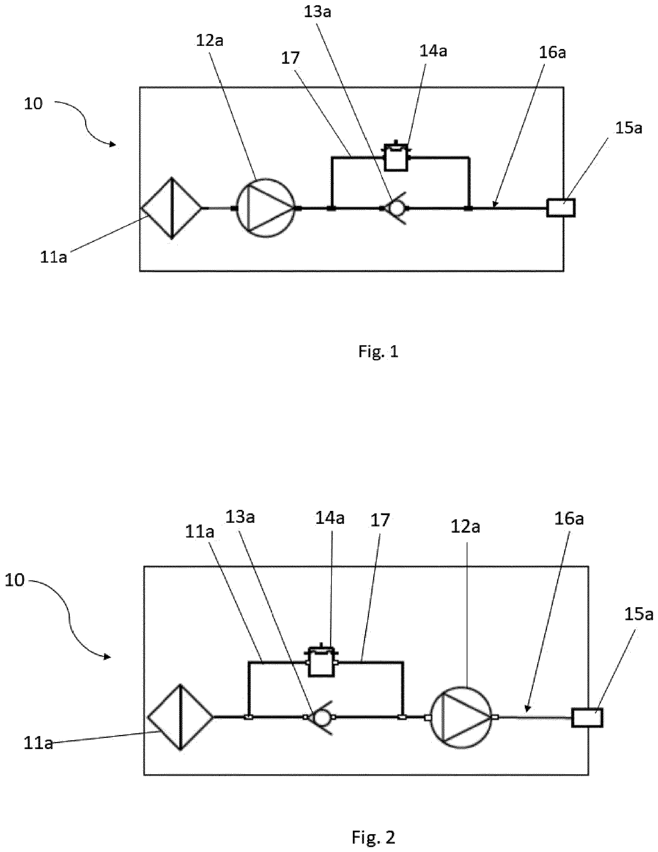

- Fig. 1: einen schematischen Aufbau einer Ausführungsform eines Beatmungsgerätes mit einem inspiratorischen Atemgasweg mit einem Bypass um ein Rückschlagventil,
- Fig. 2: eine alternative Anordnung der in Fig. 1 gezeigten Ausführungsform des Beatmungsgerätes mit dem inspiratorischen Atemgasweg mit dem Bypass um ein Rückschlagventil,

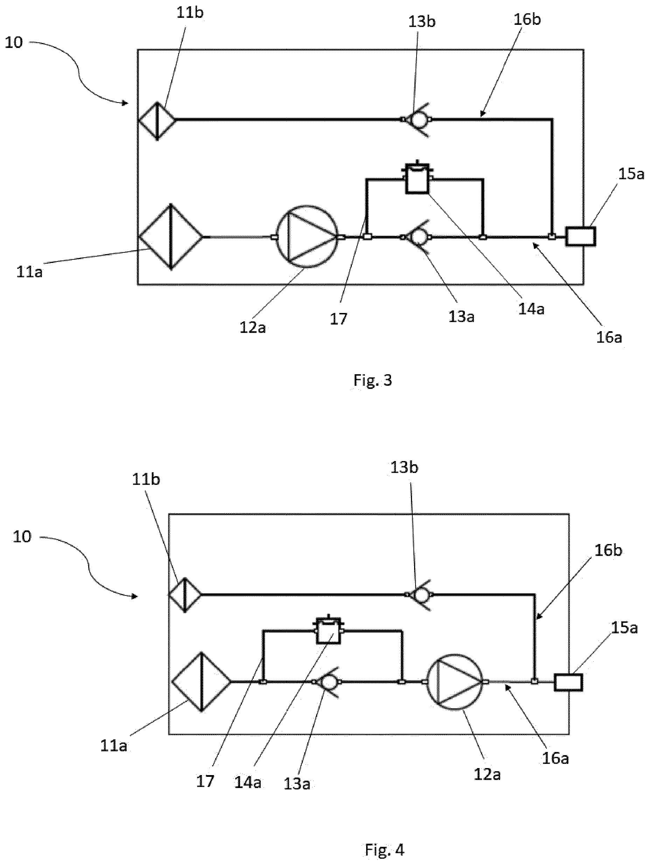

- Fig. 3: einen schematischen Aufbau einer weiteren Ausführungsform des in Fig. 1 gezeigten Beatmungsgerätes, mit dem inspiratorischen Atemgasweg mit dem Bypass und einem zweiten inspiratorischen Atemgasweg,
- Fig. 4: eine alternative Anordnung der in Fig. 2 gezeigten Ausführungsform des in Fig. 1 gezeigten Beatmungsgerätes, mit dem inspiratorischen Atemgasweg mit dem Bypass und dem zweiten inspiratorischen Atemgasweg,

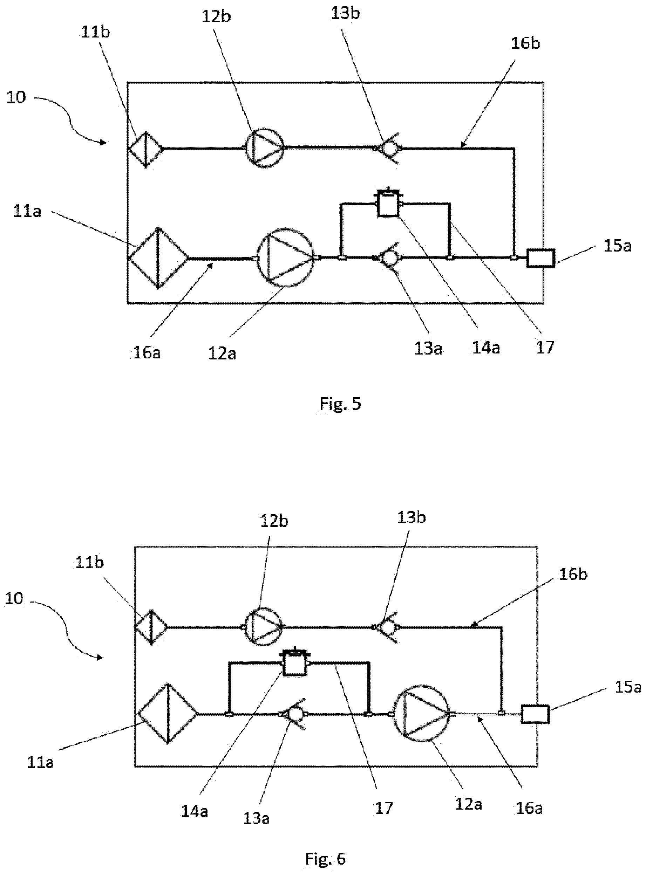

- Fig. 5: eine alternative Anordnung der in Fig. 1 und Fig. 3 gezeigten Ausführungsform des Beatmungsgerätes, mit dem inspiratorischen Atemgasweg mit dem Bypass und dem zweiten inspiratorischen Atemgasweg,
- Fig. 6: eine alternative Anordnung der in Fig. 2 und Fig. 4 gezeigten Ausführungsform des Beatmungsgerätes, mit dem inspiratorischen Atemgasweg mit dem Bypass und dem zweiten inspiratorischen Atemgasweg,

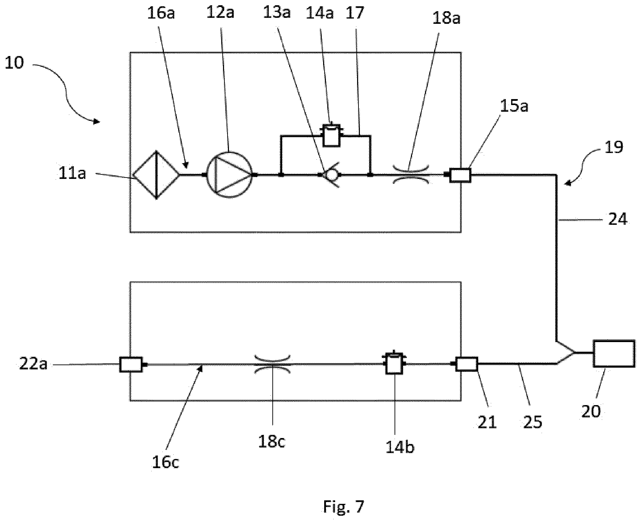

- Fig. 7: einen schematischen Aufbau einer Ausführungsform des Beatmungsgerätes mit dem inspiratorischen Atemgasweg mit dem Bypass und dem exspiratorischen Atemgasweg,

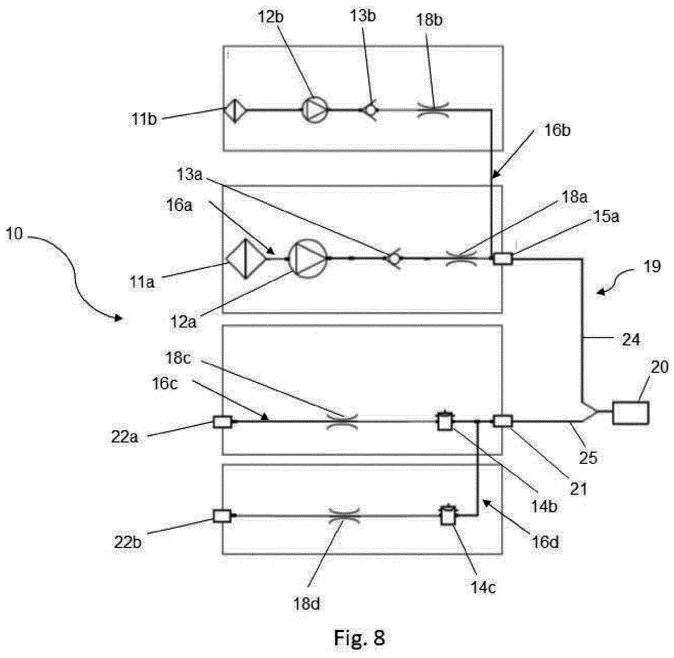

- Fig. 8: einen schematischen Aufbau einer weiteren Ausführungsform des Beatmungsgerätes mit dem inspiratorischen Atemgasweg und dem zweiten inspiratorischen Atemgasweg sowie mit einem exspiratorischen Atemgasweg und einem separaten exspiratorischen Atemgasweg,

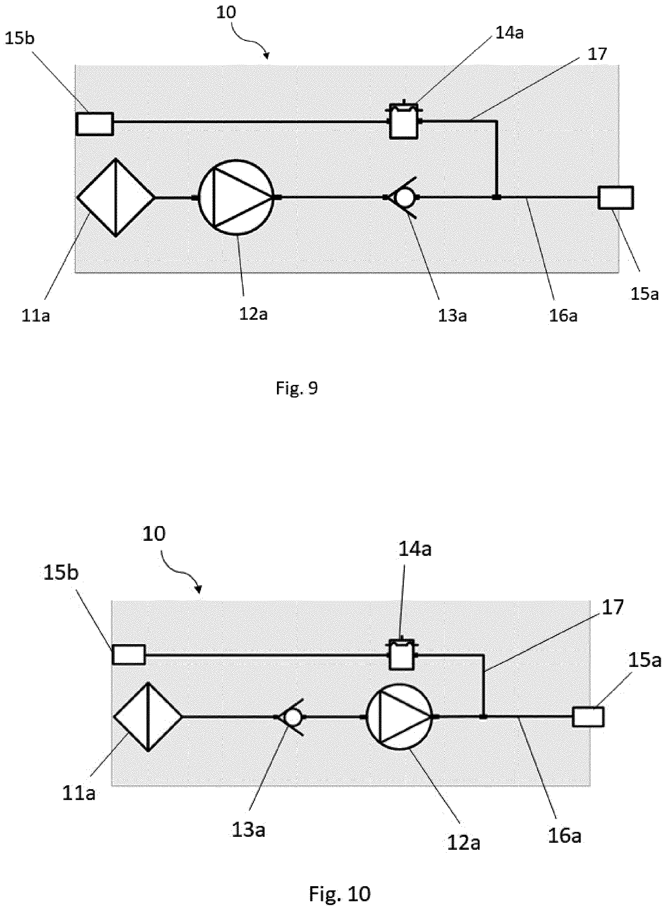

- Fig. 9: einen schematischen Aufbau einer alternativen Ausführungsform des Beatmungsgerätes mit dem inspiratorischen Atemgasweg mit einem offenen Bypass mit einem separaten Geräteausgang,
- Fig. 10: einen schematischen Aufbau einer alternativen Ausführungsform des in Fig. 9 gezeigten Beatmungsgerätes mit dem inspiratorischen Atemgasweg mit dem offenen Bypass mit dem separaten Geräteausgang,

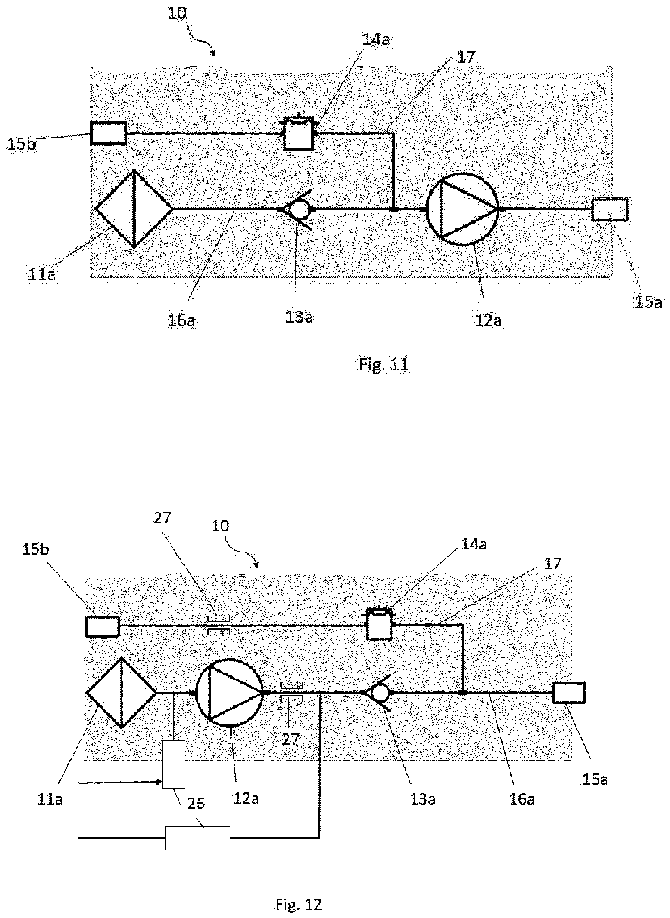

- Fig. 11: einen schematischen Aufbau einer alternativen Ausführungsform des in Fig. 9 gezeigten Beatmungsgerätes mit dem inspiratorischen Atemgasweg mit dem offenen Bypass mit dem separaten Geräteausgang,
- Fig. 12: einen schematischen Aufbau einer alternativen Ausführungsform des-in den Figuren 9 und 11 gezeigten-Beatmungsgerätes mit dem inspiratorischen Atemgasweg mit einem offenen Bypass mit einem separaten Geräteausgang.

In den Figuren weisen dieselben konstruktiven Elemente jeweils dieselben Bezugsziffern auf.

Figur 1 zeigt einen schematischen Aufbau einer Ausführungsform eines Beatmungsgerätes 10 mit einem inspiratorischen Atemgasweg 16a mit einem Bypass 17 und das Rückschlagventil 13a. Der inspiratorische Atemgasweg 16a umfasst einen Geräteeingang 11 a und einen Geräteausgang 15a. Der Geräteausgang 15a ist dabei patientennah angeordnet und der Geräteeingang 11a ist patientenfern angeordnet. Von dem Geräteeingang 11a erstreckt sich zu dem Geräteausgang 15a der Atemgasweg 16a. Der Atemgasweg 16a umfasst einen Atemgasantrieb 12a, ein Rückschlagventil 13a und ein Schaltventil 14a. Das Schaltventil 14a ist in einem Bypass 17 zu dem Rückschlagventil 13a angeordnet. Der Bypass 17 zweigt zwischen dem Atemgasantrieb 12a und dem Rückschlagventil 13a ab und mündet zwischen dem Rückschlagventil 13a und dem Geräteausgang 15a wieder in den inspiratorischen Atemgasweg 16a. Der Atemgasantrieb 12a ist zwischen dem Geräteeingang 11a und dem Rückschlagventil 13a angeordnet. Über das im Bypass 17 angeordnete Schaltventil 14a kann Atemgas in Richtung des Geräteeingangs 11a rückgeführt werden, wodurch das Rückschlagventil 13a umgangen wird.

Figur 2 zeigt eine alternative Anordnung der in Figur 1 gezeigten Ausführungsform des Beatmungsgerätes 10 mit dem inspiratorischen Atemgasweg 16a mit dem Bypass 17 um dem Rückschlagventil 13a. Der inspiratorische Atemgasweg 16a umfasst den Geräteeingang 11a und den Geräteausgang 15a. Von dem Geräteeingang 11a erstreckt sich zu dem Geräteausgang 15a der Atemgasweg 16a. Der Atemgasweg 16a umfasst das Rückschlagventil 13a, den Atemgasantrieb 12a und das Schaltventil 14a. Das Schaltventil 14a ist in dem Bypass 17 zu dem Rückschlagventil 13a angeordnet. Der Bypass 17 zweigt in dieser alternativen Anordnung zwischen dem Geräteeingang 11a und dem Rückschlagventil 13a ab und mündet zwischen dem Rückschlagventil 13a und dem Atemgasantrieb 12a wieder in den inspiratorischen Atemgasweg 16a. Der Atemgasantrieb 12a ist zwischen dem Rückschlagventil 13a und dem Geräteausgang 15a angeordnet. Über das im Bypass 17 angeordnete Schaltventil 14a kann Atemgas in Richtung des Geräteeingangs 11a rückgeführt werden, nachdem es den Atemgasantrieb 12a passiert hat.

Figur 3 zeigt einen schematischen Aufbau einer weiteren Ausführungsform des in Figur 1 gezeigten Beatmungsgerätes 10, mit dem inspiratorischen Atemgasweg 16a, dem Bypass 17 und einem zweiten inspiratorischen Atemgasweg 16b. Der inspiratorische Atemgasweg 16a erstreckt sich von dem Geräteeingang 11a zu dem Geräteausgang 15a. Der inspiratorische Atemgasweg 16a umfasst den Atemgasantrieb 12a, das Rückschlagventil 14a und das Schaltventil 14a, wobei das Schaltventil 14a in dem Bypass 17 zur Umgehung des Rückschlagventils 13a angeordnet ist. Die in Figur 3 gezeigte Ausführungsform des Beatmungsgerätes 10 weist einen zweiten inspiratorischen Atemgasweg 16b auf, der sich von einem zweiten Geräteeingang 11b zu dem gemeinsamen Geräteausgang 15a erstreckt. Der zweite inspiratorische Atemgasweg 16b mündet zwischen dem Rückschlagventil 13a und dem gemeinsamen Geräteausgang 15a in den inspiratorischen Atemgasweg 16a ein. Der zweite inspiratorische Atemgasweg 16b kann ein Rückschlagventil 13b umfassen. Über den zweiten inspiratorischen Atemgasweg 16b kann beispielsweise bei einer Blockade/Störung des inspiratorischen Atemgasweges 16a Atemgas über den separaten Geräteeingang 11b bezogen werden und dem Patienten zugeführt werden. Zusätzlich kann bei einer Blockade eines exspiratorischen Atemgasweges Atemgas durch Umgehung des Rückschlagventils 13a durch das Schaltventil 14a rückgeführt werden und dem Patienten eine Ausatmung ermöglicht werden.

Figur 4 zeigt eine alternative Anordnung der in Figur 2 gezeigten Ausführungsform des Beatmungsgerätes 10, mit dem inspiratorischen Atemgasweg 16a, dem Bypass 17 und dem zweiten inspiratorischen Atemgasweg 16b. Bei dieser alternativen Ausführungsform ist in dem inspiratorischen Atemgasweg 16a das Rückschlagventil 13a zwischen dem Geräteeingang 11a und dem Atemgasantrieb 12a angeordnet. Um das Rückschlagventil 13a ist der Bypass 17 ausgebildet, der zwischen dem Geräteeingang 11a abzweigt und zwischen dem Rückschlagventil 13a und dem Atemgasantrieb 12a wieder in den inspiratorischen Atemgasweg 16a einmündet. Der Atemgasantrieb 12a ist zwischen dem Rückschlagventil 13a und dem Geräteausgang 15a angeordnet. Der zweite inspiratorische Atemgasweg 16b mündet zwischen dem Atemgasantrieb 12a und dem gemeinsamen Geräteausgang 15a in den inspiratorischen Atemgasweg 16a ein. Der zweite inspiratorische Atemgasweg 16b kann ein Rückschlagventil 13b umfassen. Über den zweiten inspiratorischen Atemgasweg 16b kann auch bei dieser Ausführungsform beispielsweise bei einer Blockade/Störung des inspiratorischen Atemgasweges 16a Atemgas über den separaten Geräteeingang 11b bezogen werden und dem Patienten zugeführt werden. Zusätzlich kann ebenfalls bei einer Blockade eines exspiratorischen Atemgasweges Atemgas durch Umgehung des Rückschlagventils 13a durch das Schaltventil 14a rückgeführt werden und dem Patienten eine Ausatmung ermöglicht werden.

Figur 5 zeigt eine alternative Anordnung der in Fig. 1 und Fig. 3 gezeigten Ausführungsform des Beatmungsgerätes 10, mit dem inspiratorischen Atemgasweg 16a, dem Bypass 17 und dem zweiten inspiratorischen Atemgasweg 16b.

Der inspiratorische Atemgasweg 16a erstreckt sich von dem Geräteeingang 11a zu dem Geräteausgang 15a und umfasst den Atemgasantrieb 12a, das Rückschlagventil 14a und das Schaltventil 14a. Das Schaltventil 14a ist in dem Bypass 17 zur Umgehung des Rückschlagventils 13a angeordnet. Der Bypass 17 ist zwischen dem Atemgasantrieb 12a und dem Geräteausgang 15a ausgebildet. Die in Figur 5 gezeigte Ausführungsform des Beatmungsgerätes 10 weist ebenfalls den zweiten inspiratorischen Atemgasweg 16b auf, der sich von dem zweiten Geräteeingang 11b zu dem gemeinsamen Geräteausgang 15a erstreckt. Der zweite inspiratorische Atemgasweg 16b mündet zwischen dem Rückschlagventil 13b und dem gemeinsamen Geräteausgang 15a in den inspiratorischen Atemgasweg 16a ein. Der zweite inspiratorische Atemgasweg 16b weist ein Rückschlagventil 13b und einen Atemgasantrieb 12b auf, wobei der Atemgasantrieb 12b zwischen dem zweiten Geräteeingang 11b und dem Rückschlagventil 13b angeordnet ist. Über den zweiten inspiratorischen Atemgasweg 16b kann auch bei dieser Ausführungsform beispielsweise bei einer Blockade/Störung des inspiratorischen Atemgasweges 16a Atemgas über den separaten Geräteeingang 11b bezogen werden und dem Patienten zugeführt werden. Zusätzlich kann ebenfalls bei einer Blockade eines exspiratorischen Atemgasweges Atemgas durch Umgehung des Rückschlagventils 13a über das im Bypass 17 angeordnete Schaltventil 14a rückgeführt werden und dem Patienten eine Ausatmung ermöglicht werden.

Figur 6 zeigt eine alternative Anordnung der in Figur 2 und Figur 4 gezeigten Ausführungsform des Beatmungsgerätes 10, mit dem inspiratorischen Atemgasweg 16a, dem Bypass 17 und dem zweiten inspiratorischen Atemgasweg 16b. Der inspiratorische Atemgasweg 16a erstreckt sich von dem Geräteeingang 11a zu dem Geräteausgang 15a und umfasst das Rückschlagventil 14a, das Schaltventil 14a und den Atemgasantrieb 12a. Das Schaltventil 14a ist in dem Bypass 17 zur Umgehung des Rückschlagventils 13b angeordnet. Der Bypass 17 mit dem Schaltventil 14a ist zwischen dem Geräteingang 11a und dem Atemgasantrieb 12a angeordnet. Die in Figur 6 gezeigte Ausführungsform des Beatmungsgerätes 10 weist den zweiten inspiratorischen Atemgasweg 16b auf, der sich von einem zweiten Geräteeingang 11b zu dem gemeinsamen Geräteausgang 15a erstreckt. Der zweite inspiratorische Atemgasweg 16b mündet zwischen dem Atemgasantrieb 12a und dem gemeinsamen Geräteausgang 15a in den inspiratorischen Atemgasweg 16a ein. Der zweite inspiratorische Atemgasweg 16b weist ein Rückschlagventil 13b und einen Atemgasantrieb 12b auf, wobei der Atemgasantrieb 12b zwischen dem zweiten Geräteeingang 11b und dem Rückschlagventil 13b angeordnet ist.

Figur 7 einen schematischen Aufbau einer Ausführungsform des Beatmungsgerätes 10 mit dem inspiratorischen Atemgasweg 16a mit einem Bypass 17 und einem exspiratorischen Atemgasweg 16c.

Der inspiratorische Atemgasweg 16a erstreck sich von dem Geräteeingang 11a hin zu dem Geräteausgang 15a. Der inspiratorische Atemgasweg 16a umfasst dabei den Atemgasantrieb 12a sowie das Rückschlagventil 13a und das Schaltventil 14a, welches in einem Bypass 17 zu dem Rückschlagventil 13a angeordnet ist sowie eine Flussmesseinrichtung 18a. Die Flussmesseinrichtung 18a ist zwischen dem Bypass 17 und dem Geräteausgang 15a angeordnet. Über die Flussmesseinrichtung 18a kann ein Fluss bzw. ein Volumen oder ein Druck des Atemgases im Atemgasweg erfasst werden. Bei einem vorgegebenen Volumen oder Druck an Atemgas über einen vorgegebenen Zeitraum ist das Schaltventil beispielsweise mittels eines 3/2-Wegeventils schaltbar. Das Schaltventil ist in der Regel eingerichtet und ausgebildet einen PEEP (positive end-expiratory pressure) im Bereich von 0-20hPa, bevorzugt 0-15hPa einzustellen.

Der exspiratorische Atemgasweg 16c erstreckt sich von einem exspiratorischen Geräteeingang 21 zu dem exspiratorischen Geräteausgang 22a und umfasst ein Schaltventil 14b und eine Flussmesseinrichtung 18c. Die Flussmesseinrichtung 18c ist dabei zwischen dem exspiratorischen Geräteausgang 22a und dem Schaltventil 14b angeordnet. Die Flussmesseinrichtung 18c dient dabei der Erfassung eines Flusses bzw. eines Volumens oder eines Druckes des Atemgases im Atemgasweg. Basierend auf den durch die Flussmesseinrichtung 18c erfassten Werte kann eine Rückmeldung über das Volumen des durch den Patienten abgegebenen Atemgases an das Beatmungsgerät erfolgen.

An den Geräteausgang 15a ist ein Schlauchsystem 19 mit einem ersten Zweig 24 und einem zweiten Zweig 25 adaptiert. Der erste Zweig 24 führt von dem Geräteausgang 15a zu einem Patienteninterface 20. Vor dem Patienteninterface 20 führt das Schlauchsystem 19 mit einem zweiten Zweig 25 zu dem exspiratorischen Geräteeingang 21 für exspiratorisches Atemgas. Über das Schlauchsystem 19 ist ein Patienteninterface 20 mit dem inspiratorischen Zweig 16a und dem exspiratorischen Zweig 16c verbunden.

Bei der in Figur 7 gezeigten Ausführungsform des Beatmungsgerätes 10 kann ein Atemgas über den Geräteeingang 11a bezogen werden und über den Atemgasantrieb 12a über das Rückschlagventil 13a in Richtung des Geräteausgangs 15a geführt werden. Das Atemgas wird von dem Geräteausgang 15a über das den Zweig 24 des adaptierten Schlauchsystems 19 zu dem Patienteninterface 20 weitergeleitet, worüber ein Patient das Atemgas einatmen kann. Die ausgeatmete Luft des Patienten kann über den Zweig 25 des adaptierten Schlauchsystems 19 über den exspiratorischen Geräteeingang 21 dem Atemgasweg 16c zugeführt werden. Über den exspiratorischen Atemgasweg 16c wird das Atemgas hin zu dem exspiratorischen Geräteausgang 22a geführt und an die Umgebung abgegeben. Bei einer Blockade/Störung des exspiratorischen Atemgasweges 16c kann das Atemgas durch Öffnung des Schaltventils 14a in dem Bypass 17 in dem inspiratorischen Atemgasweg 16a abgeführt werden.

Figur 8 zeigt einen schematischen Aufbau einer weiteren Ausführungsform des Beatmungsgerätes 10 mit dem ersten inspiratorischen Atemgasweg 16a und dem zweiten inspiratorischen Atemgasweg 16b sowie mit dem exspiratorischen Atemgasweg 16c und einem separaten exspiratorischen Atemgasweg 16d.

Der inspiratorische Atemgasweg 16a erstreckt sich von dem Geräteeingang 11a hin zu dem Geräteausgang 15a. Der inspiratorische Atemgasweg 16a umfasst den Atemgasantrieb 12a, das Rückschlagventil 13a sowie eine Flussmesseinrichtung 18a. Die Flussmesseinrichtung 18a ist zwischen dem Rückschlagventil 13a und dem Geräteausgang 15a angeordnet. Die Flussmesseinrichtung 18a erfasst einen Fluss bzw. ein Volumen oder einen Druck des Atemgases welches über den inspiratorischen Atemgasweg 16a an den Patienten abgegeben wird.

Die in Figur 8 gezeigte Ausführungsform des Beatmungsgerätes 10 weist einen zweiten inspiratorischen Atemgasweg 16b auf, der sich von dem zweiten Geräteeingang 11b zu dem gemeinsamen Geräteausgang 15a erstreckt. Der zweite inspiratorische Atemgasweg 16b mündet zwischen der Flussmesseinrichtung und dem gemeinsamen Geräteausgang 15a in den inspiratorischen Atemgasweg 16a ein. Der zweite inspiratorische Atemgasweg 16b kann den separaten Atemgasantrieb 12b, das Rückschlagventil 13b und eine Flussmesseinrichtung 18b umfassen. Der separate Atemgasantrieb 12b ist zwischen dem zweiten Geräteeingang 11b und dem Rückschlagventil 13b angeordnet. Die Flussmesseinrichtung 18b kann ein Volumen oder Druck des inspiratorischen Atemgases in dem Atemgasweg 16b an erfassen und an das Beatmungsgerät 10 übermitteln. Über den zweiten inspiratorischen Atemgasweg 16b kann bei einer Blockade des inspiratorischen Atemgasweges 16a Atemgas über den Geräteeingang 11b bezogen und dem Patienten zugeführt werden.

Die in Figur 8 gezeigte Ausführungsform des Beatmungsgerätes 10 weist ferner den exspiratorische Atemgasweg 16c auf, der sich von dem exspiratorischen Geräteeingang 21 zu dem exspiratorischen Geräteausgang 22a erstreckt und das Schaltventil 14b und die Flussmesseinrichtung 18c umfasst. Die Flussmesseinrichtung 18c ist dabei zwischen dem exspiratorischen Geräteausgang 22a und dem Schaltventil 14b angeordnet. Die Flussmesseinrichtung 18c erfasst ein Volumen und/oder einen Druck des Atemgases, welches der Patient abgibt. Über den exspiratorischen Atemgasweg 16c ist ein von dem Patienten abgegebenes Atemgas abführbar.

Die Flussmesseinrichtungen 18a, 18b, 18c und 18d können eingerichtet sein, einen Fluss bzw. ein Volumen oder einen Druck des Atemgases in dem Atemgasweg zu erfassen und basierend auf diesem ein Schaltventil zu schalten bzw. zu öffnen und zu schließen oder eine Rückmeldung an das Beatmungsgerät 10 über den Fluss bzw. das Volumen oder den Druck des dem Patienten zugeführten Atemgases oder von dem Patienten abgegebenes Atemgases zu liefern.

Die in Figur 8 gezeigte Ausführungsform des Beatmungsgerätes 10 weist zudem einen separaten exspiratorischen Atemgasweg 16d auf. Der separate exspiratorische Atemgasweg 16d zweigt von dem exspiratorischen Atemgasweg 16c ab und erstreckt sich hin zu einem separaten Geräteausgang 22b. Der separate exspiratorische Atemgasweg 16d umfasst ein Schaltventil 14c und eine Flussmesseinrichtung 18d. Die Flussmesseinrichtung 18d erfasst einen Fluss bzw. ein Volumen oder einen Druck des Atemgases, welches der Patient abgibt. Über den separaten exspiratorischen Atemgasweg 16d ist bei einer Blockade/Störung des exspiratorischen Atemgaswegs 16c von dem Patienten abgegebenes Atemgas abführbar.

An den Geräteausgang 15a ist ein Schlauchsystem 19 mit einem ersten Zweig 24 und einem zweiten Zweig 25 adaptiert. Der erste Zweig 24 führt von dem Geräteausgang 15a zu einem Patienteninterface 20. Vor dem Patienteninterface 20 führt das Schlauchsystem 19 mit dem zweiten Zweig 25 zu dem exspiratorischen Geräteeingang 21 für exspiratorisches Atemgas. Über das Schlauchsystem 19 ist das Patienteninterface 20 mit dem inspiratorischen Atemgasweg 16a, dem zweiten inspiratorischen Atemgasweg 16b, dem exspiratorischen Atemgasweg 16c und dem separaten exspiratorischen Atemgasweg 16d verbunden.

Figur 9 zeigt einen schematischen Aufbau einer alternativen Ausführungsform des Beatmungsgerätes 10 mit dem inspiratorischen Atemgasweg 16a mit einem offenen Bypass 17 mit einem separaten Geräteausgang 15b. Der inspiratorische Atemgasweg 16a des Beatmungsgerätes 10 umfasst den Geräteeingang 11a und den Geräteausgang 15a. Zwischen dem Geräteeingang 11a und dem Geräteausgang 15a sind der Atemgasantrieb 12a, das Rückschlagventil 13a sowie das Schaltventil 14a angeordnet, wobei das Schaltventil 14a in dem Bypass 17 angeordnet ist. Der Bypass 17 ist als offener Bypass ausgebildet, der einen separaten Geräteausgang 15b umfasst. Der offene Bypass 17 zweigt von dem inspiratorischen Atemgasweg 16a zwischen dem Rückschlagventil 13a und dem Geräteausgang 15a ab und mündet in dem separaten Geräteausgang 15b. Der Atemgasantrieb 12a ist zwischen dem Geräteeingang 11 und dem Rückschlagventil 13a angeordnet. Das Schaltventil 14a kann zeitgesteuert, patientengetriggert oder basierend auf einem durch eine Flussmesseinrichtung erfassten Fluss bzw. Volumen des Atemgases schaltbar sein. Die Flussmessrichtung ist dabei zwischen dem separaten Geräteausgang 15b und dem Schaltventil 14a angeordnet sein. Über das in dem offenen Bypass 17 angeordnete Schaltventil 14a ist es möglich, bei einer Blockade des inspiratorischen Atemgaswegs 16a von dem Patienten ausgegebenes Atemgas über das Schaltventil 14a abzugeben. Durch die Anordnung des Rückschlagventils 13a zwischen dem Atemgasantrieb 12a und dem Geräteausgang 15a, ist das Rückschlagventil 13a auf der Druckseite des Beatmungsgerätes 10 angeordnet.

Figur 10 zeigt einen alternativen schematischen Aufbau einer in Fig. 9 gezeigten Ausführungsform des Beatmungsgerätes 10 mit dem inspiratorischen Atemgasweg 16a und dem offenen Bypass 17 mit dem separaten Geräteausgang 15b. Der inspiratorische Atemgasweg 16a des Beatmungsgerätes 10 umfasst dabei den Geräteeingang 11a und den Geräteausgang 15a. Zwischen dem Geräteeingang 11a und dem Geräteausgang 15a ist das Rückschlagventil 13a, der Atemgasantrieb 12a sowie das Schaltventil 14a angeordnet, wobei das Schaltventil 14a in dem Bypass 17 angeordnet ist. Der Bypass 17 ist als offener Bypass ausgebildet, der einen separaten Geräteausgang 15b umfasst. Der offene Bypass 17 zweigt von dem inspiratorischen Atemgasweg 16a zwischen dem Atemgasantrieb 12a und dem Geräteausgang 15a ab und mündet in dem separaten Geräteausgang 15b. Der Atemgasantrieb 12a ist zwischen dem Rückschlagventil 13a und dem Geräteausgang 15a angeordnet. Über das in dem offenen Bypass 17 angeordnete Schaltventil 14a ist es möglich, bei einer Blockade des inspiratorischen Atemgaswegs 16a von dem Patienten ausgegebenes Atemgas über das Schaltventil 14a abzugeben. Durch die Anordnung des offenen Bypasses 17 zwischen dem Atemgasantrieb 12a und dem Geräteausgang 15a kann Atemgas rückgeführt werden, ohne den Atemgasantrieb passieren zu müssen. Durch die Anordnung des Rückschlagventils 13a zwischen dem Geräteeingang 11a und dem Atemgasantrieb 12a, ist das Rückschlagventil 13a auf der Saugseite des Beatmungsgerätes 10 angeordnet.

Figur 11 zeigt einen alternativen schematischen Aufbau einer in den Figuren 9 und 10 gezeigten alternativen Ausführungsform des Beatmungsgerätes 10 mit dem inspiratorischen Atemgasweg 16a und dem offenen Bypass 17 mit dem separaten Geräteausgang 15b. Der inspiratorische Atemgasweg 16a des Beatmungsgerätes 10 umfasst dabei den Geräteeingang 11a und den Geräteausgang 15a. Zwischen dem Geräteeingang 11a und dem Geräteausgang 15a ist der Atemgasantrieb 12a, das Rückschlagventil 13 sowie das Schaltventil 14a, wobei das Schaltventil 14a in dem Bypass 17 angeordnet ist. Der Bypass 17 ist als offener Bypass ausgebildet, der einen separaten Geräteausgang 15b umfasst. Der offene Bypass 17 zweigt von dem inspiratorischen Atemgasweg 16a zwischen dem Rückschlagventil 13a und dem Atemgasantrieb 12a ab und mündet in dem separaten Geräteausgang 15b. Der Atemgasantrieb 12a ist zwischen dem Rückschlagventil 13a und dem Geräteausgang 15a angeordnet. Über das in dem offenen Bypass 17 angeordnete Schaltventil 14a ist es möglich, bei einer Blockade des inspiratorischen Atemgaswegs 16a von dem Patienten ausgegebenes Atemgas über das Schaltventil 14a abzugeben. Durch die Anordnung des Rückschlagventils 13a zwischen dem Geräteeingang 11a und dem Atemgasantrieb 12a, ist das Rückschlagventil 13a auf der Saugseite des Beatmungsgerätes 10 angeordnet.

Figur 12 zeigt einen schematischen Aufbau einer alternativen Ausführungsform des Beatmungsgerätes 10 mit dem inspiratorischen Atemgasweg 16a mit dem offenen Bypass 17 mit dem separaten Geräteausgang 15b. Der inspiratorische Atemgasweg 16a des Beatmungsgerätes 10 umfasst den Geräteeingang 11a und den Geräteausgang 15a. Zwischen dem Geräteeingang 11a und dem Geräteausgang 15a sind der Atemgasantrieb 12a, das Rückschlagventil 13a, eine Flussmesseinrichtung 27 sowie das Schaltventil 14a angeordnet, wobei das Schaltventil 14a in dem Bypass 17 angeordnet ist. Der Bypass 17 ist als offener Bypass ausgebildet, der einen separaten Geräteausgang 15b sowie zumindest eine Flussmesseinrichtung 27 umfasst. Der offene Bypass 17 zweigt von dem inspiratorischen Atemgasweg 16a zwischen dem Rückschlagventil 13a und dem Geräteausgang 15a ab und mündet in dem separaten Geräteausgang 15b. Der Atemgasantrieb 12a ist zwischen dem Geräteeingang 11a und dem Rückschlagventil 13a angeordnet. Das Schaltventil 14a kann zeitgesteuert, patientengetriggert oder basierend auf einen durch die in dem Bypass 17 angeordnete Flussmesseinrichtung 27 erfassten Fluss bzw. Volumen des Atemgases schaltbar sein. Die Flussmessrichtung 27 im Bypass 17 ist dabei zwischen dem separaten Geräteausgang 15b und dem Schaltventil 14a angeordnet. Über das in dem offenen Bypass 17 angeordnete Schaltventil 14a ist es möglich, bei einer Blockade des inspiratorischen Atemgaswegs 16a von dem Patienten ausgegebenes Atemgas über das Schaltventil 14a abzugeben. Durch die Anordnung des Rückschlagventils 13a zwischen dem Atemgasantrieb 12a und dem Geräteausgang 15a, ist das Rückschlagventil 13a auf der Druckseite des Beatmungsgerätes 10 angeordnet.

Die in Figur 12 gezeigte Ausführungsform zeigt ferner, dass der inspiratorische Atemgasweg 16a zusätzlich zumindest ein weiteres Ventil 26 umfasst, welches eingerichtet ist, eine Sauerstoff- oder Luft bzw. Druckluftzufuhr zu dem inspiratorischen Atemgasweg 16a zu gewährleisten. Zudem umfasst der inspiratorische Atemgasweg 16a zumindest eine Flussmesseinrichtung 27, wobei die Flussmesseinrichtung 27 zwischen dem Atemgasantrieb 12a und dem Rückschlagventil 13a angeordnet ist. Das weitere Ventil 26 kann zwischen dem Geräteeingang 11a und dem Atemgasantrieb 12a sowie zwischen der Flussmesseinrichtung 27 und dem Rückschlagventil 13a in den inspiratorischen Atemgasweg 16a einmünden. Das weitere Ventil 26 ist in der Regel ein Proportionalventil.

Bei der in der Figur 12 gezeigten Ausführungsform ist der Bereich des offenen Bypasses 17 mit dem Schaltventil 14a, der Flussmesseinrichtung 27 und dem separaten Geräteausgang 15b sowie der Bereich des inspiratorischen Atemgasweges 16a mit dem Rückschlagventil 13a und dem Geräteausgang 15a aus dem Beatmungsgerät entnehmbar und autoklavierbar. Der Bereich des inspiratorischen Atemgasweges 16a mit dem Geräteeingang 11a, dem Atemgasantrieb 12a und der Flussmesseinrichtung 27 ist derart eingerichtet und ausgebildet, dass dieser nicht mit kontaminiertem Atemgas in Kontakt kommt.

Optional können die in den Figuren 9 bis 11 gezeigten alternativen Ausführungsformen des Beatmungsgerätes 10 jeweils eine Flussmesseinrichtung umfassen, die einen Fluss bzw. ein Volumen oder einen Druck des Atemgases im Atemgasweg erfasst.

Optional sind kann das Schaltventil 14a in den alternativen Ausführungsformen, wie in den Figuren 9 bis 11 gezeigt, basierend auf dem durch die Flussmesseinrichtung erfassten Fluss bzw. Volumen schaltbar sein.

Optional können die in den Figuren 9 bis 11 gezeigten alternativen Ausführungsformen mit den in den Figuren 1 bis 8 gezeigten Ausführungsformen kombiniert werden. Alternativ kann der in den Figuren 9 bis 11 gezeigte offene Bypass 17 den geschlossenen Bypass 17 in den in den Figuren 1 bis 8 gezeigten Ausführungsformen ersetzen.

### Bezugszeichenliste

- 10: Beatmungsgerät
- 11a: Geräteeingang
- 11b: zweiter, separater Geräteeingang
- 12a: Atemgasantrieb
- 12b: Atemgasantrieb
- 13a: Rückschlagventil
- 13b: Rückschlagventil
- 14a: Schaltventil
- 14b: Schaltventil
- 14c: Schaltventil
- 14d: Schaltventil
- 15a: Geräteausgang
- 15b: separater Geräteausgang
- 16a: erster Atemgasweg
- 16b: zweiter Atemgasweg
- 16c: Atemgasweg, für den exspiratorischen Atemgasfluss
- 16d: separater exspiratorischer Atemgasweg
- 17: Bypass
- 18a: Flussmesseinrichtung
- 18b: Flussmesseinrichtung
- 18c: Flussmesseinrichtung
- 18d: Flussmesseinrichtung
- 19: Schlauchsystem
- 20: Patienteninterface
- 21: exspiratorischer Geräteeingang
- 22a: exspiratorischer Geräteausgang
- 22b: exspiratorischer Geräteausgang
- 23: Schlauchsystem
- 24: erster Zweig
- 25: zweiter Zweig
- 26: weitere Ventile, Proportionalventil
- 27: weitere Flussmesseinrichtungen

## Patentansprüche

1. Beatmungsgerät (10) mit mindestens einem inspiratorischen Geräteeingang (11a) und einem inspiratorischen Geräteausgang (15a) und einem inspiratorischen Atemgasweg (16a) zwischen dem inspiratorischen Geräteeingang (11a) und dem inspiratorischen Geräteausgang (15a), und einem exspiratorischen Atemgasweg (16c), wobei im inspiratorischen Atemgasweg (16a) ein Atemgasantrieb (12a), ein Rückschlagventil (13a) und mindestens ein Schaltventil (14a) angeordnet sind, wobei das Rückschlagventil (13a) eingerichtet ist einen Atemgasstrom über den inspiratorischen Atemgasweg in einer Richtung vom inspiratorischen Geräteausgang (15a) zum inspiratorischen Geräteeingang (11a) zu verhindern und wobei das mindestens eine Schaltventil (14a) in einem Bypass (17) zu dem Rückschlagventil (13a) angeordnet ist und/oder einen Bypass (17) um das Rückschlagventil (13a) ausbildet, wobei der Bypass (17) zu dem mindestens einen Schaltventil (14a) zwischen dem inspiratorischen Geräteeingang (11a) und dem Rückschlagventil (13a) abzweigt und zwischen dem Rückschlagventil (13a) und dem Atemgasantrieb (12a) wieder in den inspiratorischen Atemgasweg (16a) mündet, und wobei das mindestens eine Schaltventil (14a) eingerichtet ist bei einer Blockade des exspiratorischen Atemgasweges, die Abfuhr von Atemgas des Patienten über den inspiratorischen Atemgasweg durch Öffnung des mindestens einen Schaltventils (14a) zu ermöglichen, indem durch das mindestens eine Schaltventil (14a) das Rückschlagventil (13a) umgangen wird und dafür einen Atemgasstrom in einer Richtung vom inspiratorischen Geräteausgang (15a) zum inspiratorischen Geräteeingang (11a) über den Bypass (17) zumindest zeitweise zu ermöglichen.

2. Beatmungsgerät (10) nach Anspruch 1, **dadurch gekennzeichnet, dass** zwischen dem Rückschlagventil (13a) und dem inspiratorischen Geräteausgang (15a) eine Flussmesseinrichtung (18a) angeordnet ist.

3. Beatmungsgerät (10) nach zumindest einem der Ansprüche 1 oder 2, **dadurch gekennzeichnet, dass** das mindestens eine Schaltventil (14a) eingerichtet ist, den Bypass (17) zu öffnen und / oder zu schließen, wobei das mindestens eine Schaltventil (14a) automatisch oder aktiv schaltbar eingerichtet ist.

4. Beatmungsgerät (10) nach einem der Ansprüche 1 bis 3, **dadurch gekennzeichnet, dass** der Atemgasantrieb (12a) zwischen dem Rückschlagventil (13a) und dem inspiratorischen Geräteausgang (15a) angeordnet ist und eingerichtet ist, Atemgas in Richtung des inspiratorischen Geräteausgangs (15a) zu fördern

5. Beatmungsgerät (10) nach zumindest einem der voranstehenden Ansprüche, **dadurch gekennzeichnet, dass** zusätzlich zu dem Bypass (17) ein zweiter inspiratorischer Atemgasweg (16b) ausgebildet ist, der einen zweiten inspiratorischen Geräteeingang (11b) und mindestens ein weiteres Rückschlagventil (13b) umfasst und vor dem gemeinsamen inspiratorischen Geräteausgang (15a) in den ersten inspiratorischen Atemgasweg (16a) mündet.

6. Beatmungsgerät (10) nach zumindest einem der voranstehenden Ansprüche, **dadurch gekennzeichnet, dass** der exspiratorische Atemgasweg (16c) einen exspiratorischen Geräteeingang (21) und einen exspiratorischen Geräteausgang (22a) umfasst.

7. Beatmungsgerät (10) nach Anspruch 6, **dadurch gekennzeichnet, dass** von dem exspiratorischen Geräteeingang (21) der exspiratorische Atemgasweg (16c) zu dem exspiratorischen Geräteausgang (22a) führt und in dem exspiratorischen Atemgasweg (16c) ein weiteres Schaltventil (14b) und eine Flussmesseinrichtung (18c) angeordnet sind.

8. Beatmungsgerät (10) nach Anspruch 7 **dadurch gekennzeichnet, dass** zwischen dem exspiratorischen Geräteeingang (21) und dem weiteren Schaltventil (14b) ein separater weiterer exspiratorischer Atemgasweg (16d) mit einem weiteren Schaltventil (14c) und einer Flussmesseinrichtung (18d) zu einem separaten exspiratorischen Geräteausgang (22b) führt.

9. Beatmungsgerät (10) nach zumindest einem der voranstehenden Ansprüche 6 bis 8, **dadurch gekennzeichnet, dass** an den inspiratorischen Geräteausgang (15a) ein Schlauchsystem (19) adaptiert ist, das mit einem ersten Zweig (24) zu einem Patienteninterface (20) führt und vor dem Patienteninterface (20) mit einem zweiten Zweig (25) zu dem exspiratorischen Geräteeingang (21) führt.

## Claims

1. A respiratory apparatus (10) with at least one inspiratory device inlet (11a) and an inspiratory device outlet (15a) and an inspiratory respiratory gas path (16a) between the inspiratory device inlet (11a) and the inspiratory device outlet (15a) and an expiratory respiratory path (16c), wherein a respiratory gas drive (12a), a check valve (13a), and at least one switching valve (14a) are arranged in the inspiratory respiratory gas path (16a), wherein the check valve (13a) is configured to prevent a flow of respiratory gas via the inspiratory respiratory gas path in a direction from the inspiratory device outlet (15a) to the inspiratory device inlet (11a), and wherein the at least one switching valve (14a) is arranged in a bypass (17) to the check valve (13a) and/or a bypass (17) to the at least one switching valve (14a) between the inspiratory device inlet (11a) and the check valve (13a) and the respiratory gas drive (12a) opens again into the inspiratory respiratory gas path (16a), and wherein the at least one switching valve (14a) is configured to enable the discharge of respiratory gas of the patient via the inspiratory respiratory gas path when the expiratory respiratory gas path is blocked by opening the at least one switching valve (14a) in that the check valve (13a) is bypassed by the at least one switching valve (14a) and, for this, to enable a flow of respiratory gas in a direction from the inspiratory device outlet (15a) to the inspiratory device inlet (11a) via the bypass (17) at least temporarily.

2. The respiratory apparatus (10) according to claim 1, **characterized in that** a flow measuring apparatus (18a) is arranged between the check valve (13a) and the inspiratory device outlet (15a).

3. The respiratory apparatus (10) according to at least one of claims 1 or 2, **characterized in that** the at least one switching valve (14a) is configured to open and/or to close the bypass (17), wherein the at least one switching valve (14a) is configured to be switchable automatically or actively.

4. The respiratory apparatus (10) according to any one of claims 1 to 3, **characterized in that** the respiratory gas drive (12a) is arranged between the check valve (13a) and the inspiratory device outlet (15a) and is configured to convey respiratory gas in the direction of the inspiratory device outlet (15a).

5. The respiratory apparatus (10) according to at least one of the preceding claims, **characterized in that,** in addition to the bypass (17), a second inspiratory respiratory gas path (16b) is designed, which comprises a second inspiratory device inlet (11b) and at least one further check valve (13b) and opens into the first inspiratory respiratory gas path (16a) before the common inspiratory device outlet (15a).

6. The respiratory apparatus (10) according to at least one of the preceding claims, **characterized in that** the expiratory respiratory gas path (16c) comprises an expiratory device inlet (21) and an expiratory device outlet (22a).

7. The respiratory apparatus (10) according to claim 6, **characterized in that,** from the expiratory device inlet (21), the expiratory respiratory gas path (16c) leads to the expiratory device outlet (22a) and a further switching valve (14b) and a flow measuring apparatus (18c) are arranged in the expiratory respiratory gas path (16c).

8. The respiratory apparatus (10) according to claim 7, **characterized in that,** between the expiratory device inlet (21) and the further switching valve (14b), a separate further expiratory respiratory gas path (16d) with a further switching valve (14c) and a flow measuring apparatus (18d) leads to a separate expiratory device outlet (22b).

9. The respiratory apparatus (10) according to at least one of the preceding claims 6 to 8, **characterized in that** a tube system (19) that leads with a first branch (24) to a patient interface (20) and leads with a second branch (25) before the patient interface (20) to the expiratory device inlet (21) is adapted on the inspiratory device outlet (15a).

## Revendications

1. Appareil respiratoire (10) comprenant au moins une entrée d'appareil inspiratoire (11a) et une sortie d'appareil inspiratoire (15a) ainsi qu'un trajet de gaz respiratoire inspiratoire (16a) entre l'entrée d'appareil inspiratoire (11a) et la sortie d'appareil inspiratoire (15a) et un trajet de gaz respiratoire expiratoire (16c), dans lequel un entraînement de gaz respiratoire (12a), une soupape antiretour (13a) et au moins une soupape de commande (14a) sont disposés dans le trajet de gaz respiratoire inspiratoire (16a), dans lequel la soupape antiretour (13a) est configurée pour empêcher un flux de gaz respiratoire dans une direction allant de la sortie d'appareil inspiratoire (15a) à l'entrée d'appareil inspiratoire (11a) par le biais du trajet de gaz respiratoire inspiratoire et dans lequel l'au moins une soupape de commande (14a) est disposée dans une dérivation (17) vers la soupape antiretour (13a) et/ou une dérivation (17) vers l'au moins une soupape de commande (14a) débouche de nouveau sur le trajet de gaz respiratoire inspiratoire (16a) entre l'entrée d'appareil inspiratoire (11a) et la soupape antiretour (13a) et l'entraînement de gaz respiratoire (12a), et dans lequel l'au moins une soupape de commande (14a) est configurée pour permettre, lors d'un blocage du trajet de gaz respiratoire expiratoire, l'évacuation de gaz respiratoire du patient par le biais du trajet de gaz respiratoire inspiratoire par ouverture de l'au moins une soupape de commande (14a), la soupape antiretour (13a) étant ainsi contournée grâce à la soupape de commande (14a), et pour permettre à cet effet un flux de gaz respiratoire au moins temporairement dans une direction allant de la sortie d'appareil inspiratoire (15a) vers l'entrée d'appareil inspiratoire (11a) par le biais de la dérivation (17).

2. Appareil respiratoire (10) selon la revendication 1, **caractérisé en ce qu'**un débitmètre (18a) est disposé entre la soupape antiretour (13a) et la sortie d'appareil inspiratoire (15a).

3. Appareil respiratoire (10) selon l'une au moins des revendications 1 ou 2, **caractérisé en ce que** l'au moins une soupape de commande (14a) est configurée pour ouvrir et/ou fermer la dérivation (17), dans lequel l'au moins une soupape de commande (14a) est configurée de manière à pouvoir être commutée automatiquement ou activement.

4. Appareil respiratoire (10) selon l'une des revendications 1 à 3, **caractérisé en ce que** l'entraînement de gaz respiratoire (12a) est disposé entre la soupape antiretour (13a) et la sortie d'appareil inspiratoire (15a) et configuré pour transporter du gaz respiratoire vers la sortie d'appareil inspiratoire (15a).

5. Appareil respiratoire (10) selon l'une au moins des revendications précédentes, **caractérisé en ce qu'**un deuxième trajet de gaz respiratoire inspiratoire (16b) est réalisé en plus de la dérivation (17), lequel comporte une entrée d'appareil inspiratoire (11b) et au moins une soupape antiretour supplémentaire (13b) et débouche sur le premier trajet de gaz respiratoire inspiratoire (16a) en amont de la sortie d'appareil inspiratoire (15a) commune.

6. Appareil respiratoire (10) selon l'une au moins des revendications précédentes, **caractérisé en ce que** le trajet de gaz respiratoire expiratoire (16c) comporte une entrée d'appareil expiratoire (21) et une sortie d'appareil expiratoire (22a).

7. Appareil respiratoire (10) selon la revendication 6, **caractérisé en ce qu'**à partir de l'entrée d'appareil expiratoire (21), le trajet de gaz respiratoire expiratoire (16c) mène vers la sortie d'appareil expiratoire (22a) et une soupape de commande supplémentaire (14b) ainsi qu'un débitmètre (18c) sont disposés sur le trajet de gaz respiratoire expiratoire (16c).

8. Appareil respiratoire (10) selon la revendication 7, **caractérisé en ce qu'**entre l'entrée d'appareil expiratoire (21) et la soupape de commande supplémentaire (14b), un trajet de gaz respiratoire expiratoire supplémentaire séparé (16d) doté d'une soupape de commande supplémentaire (14c) et d'un débitmètre (18d) mène vers une sortie d'appareil expiratoire séparée (22b).

9. Appareil respiratoire (10) selon l'une au moins des revendications 6 à 8,
**caractérisé en ce qu'**un système de tuyau (19) est adapté à la sortie d'appareil inspiratoire (15a), lequel mène vers une interface patient (20) avec une première branche (24) et vers l'entrée d'appareil expiratoire (21) avec une deuxième branche (25) en amont de l'interface patient (20).
